# FASCICULE DE BREVET EUROPEEN

(11) **EP 0 624 371 B1**
(45) Date de publication et mention de la délivrance du brevet: **31.05.2000**
(21) Numéro de dépôt: 94420126.8
(22) Date de dépôt: 19.04.1994
(51) Int. Cl.: A61K 31/60, A61K 9/50

(54) **Microcapsules pour la libération contrôlée d'acide acétylsalicylique**
Microkapseln zur verzögerten Freigabe von Acetylsalicylsäure
Microcapsules for controlled release of acetylsalicylic acid

(30) Priorité: 19.04.1993 FR 9304560
(43) Date de publication de la demande: 17.11.1994
(73) Titulaire: FLAMEL TECHNOLOGIES, Société Anonyme, 69693 Venissieux Cédex (FR)
(72) Inventeur: Burguiere, Olga, F-34170 Castelnau le Lez (FR); Yassine, Ahmad, F-34080 Montpellier (FR); Selles, Jean-Philippe, F-34000 Montpellier (FR)
(74) Mandataire: Fleurance, Raphael

(56) Documents cités:
- EP-A- 0 207 041
- EP-A- 0 411 590
- EP-A- 0 413 120

## Description

L'invention concerne des microcapsules pour la libération contrôlée de l'acide acétylsalicylique en milieu gastro-intestinal. Par libération contrôlée de l'acide acétylsalicylique, on entend le contrôle de la cinétique de cette libération tout au long du tractus gastro-intestinal.

L'invention concerne, également, un procédé de préparation des microcapsules ci-dessus.

Les objectifs pharmaceutiques visés par l'invention sont, notamment, l'inhibition sélective de la cyclooxygénase spécifique de la production de thromboxane, à l'exclusion des autres prostaglandines (prostacycline) permettant d'optimiser l'inhibition de l'agrégation plaquettaire, en vue de la prévention et/ou du traitement des maladies et des risques cardio-vasculaires.

Dans ce qui suit, on utilisera la dénomination commerciale "aspirine" ou l'abréviation "ASA" pour désigner l'acide acétylsalicylique.

La recherche et la mise au point de nouvelles formes à libération contrôlée font l'objet depuis de nombreuses années d'un intérêt constant dans l'industrie pharmaceutique. En effet, de manière générale, le contrôle de la cinétique de libération d'un principe actif permet de maintenir celui-ci à un taux plasmatique stable pendant une durée souhaitée, tout en diminuant les effets secondaires associés à une libération brutale et massive de ce principe actif.

Les formes à libération contrôlée sont particulièrement avantageuses dans le cas de certains principes actifs, tels que l'aspirine, dans la mesure où elles offrent, outre les avantages qui viennent d'être décrits, celui de permettre une meilleure tolérance locale et celui de modifier les propriétés pharmacologiques et pharmacocinétiques de ces principes actifs.

S'agissant de l'aspirine, ses utilisations comme analgésique, anti-pyrétique et anti-inflammatoire sont bien connues. Les propriétés de l'aspirine comme anti-agrégant plaquettaire ont été mises en évidence plus récemment. L'aspirine agit sur l'agrégation plaquettaire en inhibant la cyclooxygénase, qui catalyse, au niveau des plaquettes, la transformation de l'acide arachidonique en thromboxane, qui est un puissant vasoconstricteur et un stimulateur de l'agrégation plaquettaire.

L'inhibition de la cyclooxygénase plaquettaire est le résultat de son acétylation par l'aspirine. Cette acétylation s'effectue au niveau du foie, lors du premier passage hépatique.

Ce mécanisme est cependant rapidement saturable : l'aspirine, qui n'a pas été désacétylée au niveau du foie, passe dans la circulation générale et provoque l'acétylation de la cyclooxygénase des autres cellules et, notamment, de celles de l'endothélium vasculaire et gastrique.

Or, la cyclooxygénase de l'endothélium vasculaire et gastrique catalyse la formation de prostacycline qui, contrairement au thromboxane, est un vasodilatateur, un anti-agrégant plaquettaire et un cytoprotecteur. L'inhibition de la cyclooxygénase vasculaire et gastrique entraîne donc l'inhibition de la prostacycline et d'autres prostaglandines (PGE), ce qui va à l'encontre, non seulement de l'effet souhaité sur l'agrégation plaquettaire, mais entraîne également des effets secondaires de l'aspirine sur les muqueuses gastro-intestinales. Ce phénomène d'inhibition aveugle des différentes prostaglandines de l'organisme est communément dénommé dilemme de l'aspirine. Il est bien connu des scientifiques et a été largement décrit par la littérature.

Il est donc primordial de favoriser l'action directe de l'aspirine, au niveau de la circulation portale, sur les plaquettes sanguines et de minimiser, au contraire, son effet sur les cyclooxygénases des parois vasculaires en réduisant au minimum le taux d'aspirine circulant au niveau systémique.

Les formulations connues jusqu'à présent, comme par exemple celles décrites dans FR-A-2 539 995, ne permettent pas d'obtenir un tel résultat, la libération de l'aspirine étant trop rapide.

La demande de brevet FR-A-2 539 995 décrit des microgranules contenant de l'aspirine, conçues pour améliorer sa tolérance gastrique et augmenter la durée d'imprégnation médicamenteuse. Ces microgranules sont formulés pour libérer la totalité de leur principe actif en milieu duodénal et en 4 heures.

L'aspirine est fixée sur des grains support d'environ 500 à 600 µm de diamètre par enrobages successifs : une couche d'aspirine dans un mélange de polyvidone-excipient et de phtalate d'éthyle, et une couche de polymère anionique de l'acide méthacrylique, jusqu'à obtention de la cinétique de libération recherchée.

Les formes galéniques décrites dans FR-A-2 539 995, qui libèrent 100 % du principe actif en 4 heures, sont adaptées aux utilisations habituelles de l'aspirine (analgésique, antipyrétique, anti-inflammatoire), mais pas à son administration comme anti-agrégant plaquettaire.

Le dilemme de l'aspirine, bien connu et évoqué ci-dessus, tout d'abord a été appréhendé dans la demande de brevet EP 0 411 590. Face à ce dilemme, l'invention proposée dans cette demande consiste en une préparation pharmaceutique anti-agrégante plaquettaire, constituée par des microcapsules comprenant un noyau interne en ASA, recouvert d'une couche d'enrobant (e. g. copolymère acrylique/méthacrylique), elle-même revêtue d'une couche extérieure d'aspirine. Avec cette préparation, l'absorption d'ASA se déroule en deux vagues successives de 80-120 mg et 180-220 mg d'ASA.

Cette demande de brevet ne donne aucun fait expérimental montrant l'efficacité de la préparation dans la résolution du dilemme :
- inhibition du thromboxane,
- épargne de la prostacycline et d'autres prostaglandines,
- tolérance gastrique.

Les quantités d'ASA, libérées à chaque vague, restent trop importantes sur un temps trop court et saturent le système hépatique de désacétylation de l'aspirine.

Une autre proposition de l'art antérieur, relative à des formes galéniques d'aspirine inhibant le thromboxane sans diminuer le taux de prostacycline circulante, est décrite dans la demande de brevet EP 0 377 439. Ces formes galéniques sont des granules d'aspirine enrobés à l'aide d'une composition d'enrobage comprenant un copolymère acrylate/méthacrylate, de l'hydroxypropylcellulose et du chlorure de sodium. L'enrobant ainsi réalisé représente 10 à 35 % en poids sec des granules. Ces formes galéniques fournissent une cinétique de libération de 5 à 15 mg/heure, à des doses de 40 mg à 120 mg et pour des durées de 8 heures.

De manière logique, l'invention décrite dans cette demande de brevet EP 0 377 439 se propose, somme toute, de remédier au dilemme de l'aspirine en diminuant les doses d'ASA pour une durée de libération limitée à 8 heures. C'est insuffisant, puisque cela ne représente qu'une couverture thérapeutique de seulement un tiers du temps nécessaire pour l'inhibition totale du thromboxane pendant 24 heures.

Ces formes galéniques, présentées comme utiles dans le traitement des affections occlusives vasculaires chez l'homme, ne sont pas satisfaisantes. En effet, les résultats, obtenus lors d'une importante étude chez des volontaires sains, montrent que l'inhibition du thromboxane sérique est de 85 % avec une forme dosée à 50 mg et de 90 % avec la forme dosée à 75 mg, le placébo donnant lui-même une inhibition d'environ 10 %. La mesure du thromboxane urinaire donne, respectivement, des valeurs d'inhibition de 60 et 70 %.

Or, pour être efficace pharmacologiquement sur l'agrégation plaquettaire, l'inhibition du thromboxane doit être supérieure à 95 %. Par ailleurs, une inhibition de 100 % du thromboxane peut ne pas être suffisante pour empêcher une agrégation plaquettaire induite par le collagène et l'Adénosine Diphosphate. Enfin, la regénération quotidienne des plaquettes (1/6 à 1/10ème de la masse initiale chez des sujets sains, 1/4 chez des sujets à risque) et la production du thromboxane de régénération rapide par les mégacaryocytes laisse les individus, prenant une prise quotidienne d'aspirine conventionnelle, très largement en dessous du pourcentage d'inhibition minimal du thromboxane (95 %) avec en plus l'impossibilité de corriger l'effet agrégant par la présence de prostacycline vasculaire antiagrégante, elle-même inhibée à 25 %. Les formes galéniques selon la demande de brevet EP 0 377 439 ne résolvent donc pas le dilemme de l'aspirine.

En outre, elles ne sont pas adaptées à la prévention des infarctus du myocarde. En effet, la fréquence de tels accidents cardiaques augmente entre 4 et 8 heures du matin et entre 18 h et 22 heures. Ce phénomène est imputable à une hyperagrégabilité matinale et vespérale. Il est donc primordial d'avoir de l'ASA dans la veine porte pendant tout le nycthémère, afin que le thromboxane soit inhibé ; ce que ne permettent pas les formes galéniques décrites dans la demande de brevet EP 0 377 439.

Dans un tel état de la technique, l'un des objectifs essentiels de l'invention est de fournir une forme galénique à base d'ASA :
- active notamment, comme anti-agrégant plaquettaire pendant 24 heures, afin d'avoir une seule prise par jour, assurant une totale couverture thérapeutique pendant 24 heures, ce qui permet, en outre, d'abaisser le coût total du traitement,
- qui apporte une solution satisfaisante au dilemme de l'aspirine par sa sélectivité biochimique sur le thromboxane, pour atteindre une efficacité thérapeutique maximale,
- qui soit parfaitement tolérée par l'organisme,
- et qui soit préparable industriellement, de façon simple, rapide et économique.

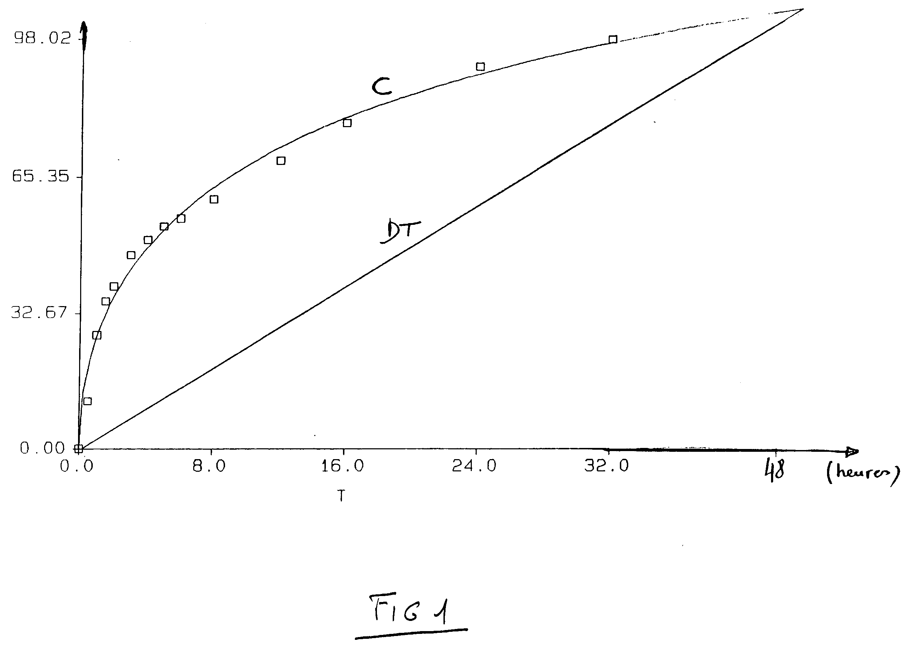

C'est ainsi que la Demanderesse a eu le mérite de mettre en évidence, après de nombreux essais et recherches, que la forme pharmaceutique ad hoc, au regard des spécifications visées ci-dessus, est constituée, avantageusement, par des particules d'ASA enrobées, de coupe granulométrique soigneusement sélectionnée et de structure telle que l'absorption in vivo de l'ASA se déroule selon un profil cinétique caractéristique s'étendant sur au moins 24 heures, pour une dose comprise entre 75 et 320 mg, ledit profil étant représenté par la courbe C sur la **fig. 1** annexée.

En conséquence, la présente invention a pour objet des microcapsules pour la libération contrôlée d'ASA en milieu gastro-intestinal, caractérisées en ce qu'elles sont constituées par des particules d'acide acétylsalicylique de taille comprise entre 100 et 1 000 µm, enrobées et conçues de telle sorte que, ingérées per os en une seule prise d'une dose D d'ASA comprise entre 75 et 320 mg, elles induisent une cinétique d'absorption d'ASA moyenne in vivo chez l'homme, s'étendant au moins sur 24 heures, ladite absorption d'ASA étant :
- inférieure ou égale à 10 % de la fraction absorbée de D à un temps **t** après l'ingestion, égal à 0,4 heure,
- inférieure ou égale à 50 % en poids de la fraction absorbée de D à **t** = 3,9 heures,
- et inférieure ou égale à 90 % en poids de la fraction absorbée de D à **t** = 23 heures,
   **t** étant donné à ± 10 % près.

Ces dispositions avantageuses sont particulièrement surprenantes et inattendues, dans la mesure où l'EP-A-0 377 439 enseigne que les formes fortement dosées connues ne conviennent pas et se propose de remédier à cela en fournissant un système faiblement dosé de libération contrôlée d'ASA, sur 8 heures. Il n'était donc pas, a priori, évident de s'intéresser, à nouveau, aux formes plus fortement dosées et ce, d'autant plus que l'on savait à quel point ces dernières pouvaient avoir une influence néfaste vis-à-vis de la prostacycline.

Selon un mode préféré de réalisation de l'invention, l'absorption se déroule sur une période comprise entre 24 et 48 heures, de la façon suivante :
- 10 % de la fraction absorbée de D à **t** compris entre 0,4 et 5 heures,
- 50 % de la fraction absorbée de D à **t** compris entre 3,9 et 25 heures,
- 90 % de la fraction absorbée de D à **t** compris entre 23 et 45 heures.

La courbe C de la **fig. 1** représente la limite supérieure du profil d'absorption in vivo de l'ASA, induite par les microcapsules selon l'invention, en fonction du temps, à une dose de 320 mg.

Cette absorption est exprimée en % absorbé par rapport à la fraction absorbée de la dose initiale D. Cette courbe C est obtenue par l'analyse classique de déconvolution (Milo GIBALDI et D. PERRIER, Pharmacokinetics, 2nd Ed., New York, Marcel Dekker Inc., 1983, p. 145-167) à partir des courbes moyennes des concentrations plasmatiques, en fonction du temps, après administration orale de 350 mg d'équivalents ASA d'ASPEGIC® (forme de référence témoin) et de 320 mg d'équivalents ASA de microcapsules selon l'invention sous forme de gélules.

La molécule traceuse retenue pour les concentrations plasmatiques, en fonction du temps, est, dans ce cas, nécessairement l'acide salicylique (SA), métabolite de l'ASA. Les concentrations plasmatiques en SA sont déterminées par HPLC.

Les points critiques à 0,4, 3,9 et 23 h donnés ci-dessus, dans la définition des microcapsules de l'invention, se retrouvent, bien entendu, sur cette courbe.

Au-delà de cette courbe C, le mécanisme hépatique de désacétylation de l'ASA est saturé. Il faut considérer que tous les profils d'absorption in vivo d'ASA contenus dans l'aire sous la courbe C relèvent de l'invention.

Conformément au mode préféré de réalisation de l'invention, les microcapsules présentent un profil d'absorption d'ASA in vivo contenu dans l'aire comprise entre la courbe C et la droite théorique DT conduisant à 100 % d'absorption en 48 heures.

Cette courbe C d'absorption in vivo, qui constitue l'une des caractéristiques essentielles de l'invention, est déterminante quant au résultat visé et atteint, d'inhibition maximale du thromboxane et d'inhibition minimale de la prostacycline et autres prostaglandines.

Il est important de noter que ces résultats remarquables sont obtenus tout en respectant les contraintes de tolérance, notamment gastrique, ainsi que de faisabilité et de rentabilité industrielle de la préparation des microcapsules.

La cinétique de libération de l'aspirine varie en fonction de la granulométrie des particules d'aspirine à encapsuler.

Par exemple, des microcapsules selon l'invention, obtenues à partir de particules d'aspirine de taille égale à 100 µm, comprenant 80 % d'aspirine et 20 % de matériau d'enrobage, libèrent, in vitro, 40 à 50 % d'aspirine au bout de 5 heures, 80% au bout de 10 heures, 90% au bout de 16 heures et la totalité au bout de 24 heures, en milieu gastro-intestinal.

D'autres microcapsules selon l'invention, à libération plus lente, peuvent, par exemple, être obtenues à partir de particules d'aspirine de taille moyenne comprise entre 315 et 400 µm, comprenant 80 % d'aspirine et 20 % de matériau d'enrobage, ces microcapsules libérant, in vitro, 35 % de l'aspirine au bout de 5 heures, 60 % au bout de 10 heures et la totalité au bout de 24 heures en milieu gastro-intestinal.

Selon une modalité préférée de l'invention, les particules d'ASA, utilisées pour l'enrobage, ont une taille comprise entre 250 et 800 µm, de préférence entre 300 et 500 µm.

Avantageusement, l'enrobant représente 5 à 50 % en poids, de préférence 10 à 40 % en poids et, plus préférentiellement encore, de 10 à 35 % en poids par rapport à la masse totale des microcapsules.

L'enrobant forme, bien entendu, l'une des pièces maîtresses de la présente invention, étant précisé que la courbe d'absorption in vivo décrite supra constitue un gabarit utile à l'homme du métier pour déterminer, de façon relativement aisée, e. g. par tâtonnements, les caractéristiques techniques d'enrobage, en particulier la nature de l'enrobant, qui permettent d'atteindre l'effet pharmacologique visé.

Avantageusement, l'enrobant est constitué par une composition d'enrobage comprenant :
- au moins un polymère filmogène (P₁) insoluble en milieu gastro-intestinal,
- au moins un polymère (P₂) soluble dans l'eau,
- au moins une charge lubrifiante solide,
- et au moins un plastifiant hydrophobe.

Une autre originalité de la présente invention s'exprime au travers de la susdite composition d'enrobage qui consiste en une sélection non arbitraire de quatre composés dont les fonctionnalités se combinent pour l'obtention du résultat visé par l'invention.

De préférence, la composition d'enrobage se définit quantitativement de la façon suivante, exprimée en % en poids sur sec :
- P₁ : 60 à 85, de préférence 70 à 80 %,
- P₂ : 2 à 20, de préférence 5 à 15 %,
- agent lubrifiant : 2 à 20, de préférence 8 à 20 %,
- plastifiant : 2 à 20, de préférence 5 à 15 %.

Selon une modalité avantageuse de l'invention, le polymère filmogène P₁ est soluble dans au moins un solvant organique de température d'ébullition comprise entre 35 et 120° C.

Avantageusement, P₂ est un polymère hydrosoluble, également soluble dans un solvant de P₁.

Il est, par ailleurs, préférable que la charge lubrifiante solide soit insoluble dans l'eau et dans les solvants de P₁.

Suivant une variante de définition quantitative de l'enrobant conforme à l'invention, ce dernier comprend de 10 à 30 parties en poids du dérivé polymère P₁, 1 à 3 parties en poids du dérivé polymère P₂, 2 à 4 parties en poids d'une charge lubrifiante solide et 1 à 3 parties en poids d'un plastifiant.

Selon une caractéristique remarquable de l'invention, P₁ est sélectionné parmi les produits suivants : la zéine, l'éthylcellulose, le chlorure de vinyle, l'acétate de vinyle et/ou ses copolymères, les copolymères à base d'acrylate et/ou de méthacrylate d'éthyle et/ou de méthyle comme, par exemple, les produits commercialisés sous la marque EUDRAGIT® RL et/ou RS et les mélanges de tous ces produits.

Sans que cela ne soit limitatif, il est à souligner que l'éthylcellulose convient particulièrement bien à titre de composé P₁.

En ce qui concerne P₂, il est sélectionné, de préférence, parmi les produits suivants :
- la polyvinylpyrrolidone,
- les dérivés cellulosiques solubles dans l'eau, tels que l'hydroxypropylcellulose, l'hydroxypropylméthylcellulose, l'hydroxyméthyléthylcellulose et la méthylcellulose,
- les copolymères de l'acétate de vinyle et de l'acide crotonique,
- les copolymères de l'anhydride maléique et du méthylvinyléther,
- ainsi que les dérivés et les mélanges de tous les susdits produits.

En général, c'est la polyvinylpyrrolidone que l'on préfère associer à P₁ pour la mise en oeuvre de l'enrobage selon l'invention.

Pour compléter celui-ci, on choisit la charge lubrifiante solide parmi les composés suivants : sels alcalinoterreux de l'acide stéarique, les silicates de magnésium, le kaolin, le talc, la silice et leurs mélanges.

Le dernier constituant de la composition selon l'invention est le plastifiant constitué par au moins l'un des produits suivants :
- les stéarates de glycol, tels que le glycérol, le propylèneglycol ou la triacétine,
- les citrates,
- les phtalates, tels que par exemple les phtalates de diméthyle, diéthyle ou dibutyle,
- les esters de l'alcool cétylique, tels que, notamment, le palmitate de cétyle,
- les sébaçates,
- les tartrates,
- l'huile de ricin,
- la cutine,
- les résines synthétiques, comme par exemple le Cérit®.

La charge lubrifiante solide et le plastifiant préférés sont, respectivement, le stéarate de magnésium et l'huile de ricin.

A titre d'exemple de composition d'enrobage plus volontiers mise en oeuvre, on peut citer celle comprenant : éthylcellulose (P₁),/polyvinylpyrrolidone (P₂)/stéarate de magnésium/huile de ricin, présents, respectivement, dans les teneurs relatives préférées suivantes :
- 74 ± 2,
- 8 ± 2,
- 10 ± 2,
- 8 ± 2 %,
   en poids sur sec.

Cette composition d'enrobage constitue l'une des spécificités originales de la présente invention. Elle est caractérisée par une combinaison intime des quatre composés indiqués ci-dessus.

Pour prévenir les problèmes de mottage des particules enrobées constituant les microcapsules de l'invention, on prévoit, avantageusement, de leur adjoindre au moins un agent anti-agglomérant formé, de préférence, par du talc, de la silice colloïdale ou par un mélange des deux.

De façon générale, l'enrobage des particules d'ASA selon l'invention est obtenu par pulvérisation de la combinaison intime formant l'enrobant, en suspension dans un solvant ou un mélange de solvants organiques.

Le procédé d'enrobage, qui constitue un autre objet de l'invention, s'inscrit dans le cadre des techniques de micro-encapsulation, dont les principales sont résumées dans l'article de C. DUVERNEY et J. P. BENOIT dans "L'actualité chimique", décembre 1986. Plus précisément, la technique considérée est la micro-encapsulation par pelliculage.

De préférence, ce procédé consiste essentiellement à :
a) - préparer la composition d'enrobage par mélange de P₁, P₂, de l'agent lubrifiant et du plastifiant dans un système solvant,
b) - appliquer le mélange composition/système solvant sur des particules d'acide acétylsalicylique,
c) - sécher les microcapsules ainsi obtenues,
d) - et éventuellement mélanger ces dernières avec au moins un agent anti-agglomérant.

Les solvants convenables pour entrer dans la composition du système solvant sont, par exemple, des cétones, des esters, des solvants chlorés, des alcools, de préférence aliphatiques, des alcanes ou des mélanges d'entre eux.

Avantageusement, ce sont des composés en C₁-C₆, étant précisé que l'acétone, la méthyléthylcétone, le méthanol, l'éthanol, l'isopropanol, le cyclohexane et le chlorure de méthylène sont particulièrement préférés.

Pour aller plus dans le détail de la méthodologie d'enrobage susceptible d'être mise en oeuvre conformément à l'invention, on peut préciser que l'application du mélange composition d'enrobage/système solvant s'opère par pulvérisation sur les particules d'ASA mises en mouvement, de préférence par agitation mécanique ou par soufflage (fluidisation).

Pour obtenir des microcapsules selon l'invention, possédant la cinétique d'absorption souhaitée, il est nécessaire d'encapsuler des particules d'ASA de taille moyenne comprise entre 75 et 500 µm, de préférence entre 300 et 500 µm pour une dose D comprise entre 75 et 320 mg.

Selon un mode préféré de mise en oeuvre du procédé, conforme à l'invention, de microencapsulation de particules d'aspirine, on prévoit les étapes suivantes :
a₁) - préparer tout d'abord un mélange comprenant de 10 à 30 parties en poids d'un polymère filmogène P₁ et 1 à 3 parties en poids d'un plastifiant pour 1 à 3 parties en poids d'un polymère hydrosoluble P₂ en solution, soit dans un mélange acétone/alcanol tel que le rapport acétone/alcanol soit compris entre 50/50 et 70/30 (v/v), soit dans un solvant choisi parmi le cyclohexane, le toluène, le tétrachlorure de carbone, le chloroforme ou le chlorure de méthylène;
a₂) - mettre en suspension, dans la solution préparée à l'étape précédente, 2 à 4 parties en poids d'agent lubrifiant, sur la base de 1 à 3 parties en poids de polymère vinylique P₂,
b) - pulvériser le mélange résultant sur les microparticules de principe actif, en lit fluidisé,
c) - sécher les microcapsules à la fin de la pulvérisation en lit fluidisé puis à l'étuve,
d) - mélanger les microcapsules ainsi obtenues avec 0,5 à 2 parties en poids d'agent anti-adhérent, sur la base de 1 à 3 parties en poids de polymère vinylique P₂.

De préférence, on additionne lors de l'étape b), soit le même alcanol, de manière à obtenir un rapport acétone/alcanol de 60/40 (v/v), soit le même solvant.

Dans la présente description, on entend par alcanol les alcools aliphatiques ayant de 1 à 6 atomes de carbone, l'isopropanol étant préféré.

Selon un mode de mise en oeuvre préféré du procédé selon l'invention, les microcapsules sont séchées, après l'enrobage.

Les microcapsules décrites ci-dessus, et éventuellement obtenues par le procédé également exposé ci-dessus, peuvent être utilisées pour la préparation de nouvelles formes galéniques d'aspirine, présentant une sélectivité biochimique d'inhibition du thromboxane par rapport aux autres prostaglandines, en particulier pour la préparation de nouvelles formes galéniques utiles comme anti-agrégant plaquettaire et/ou, plus précisément, pour la préparation de nouvelles formes galéniques actives dans la prévention et/ou le traitement des maladies et des risques cardio-vasculaires.

La présente invention concerne, en outre, ces nouvelles formes galéniques en tant que telles, originales dans leur structure, leur présentation et leur composition. Avantageusement, celles-ci se présentent sous forme de comprimés, de poudres ou de gélules dosés à 20 à 500 mg, de préférence à 50 à 400 mg et, plus préférentiellement encore, à 75 à 320 mg. De telles formes galéniques sont, de préférence, administrées per os par doses journalières uniques, comprenant chacune entre 75 et 320 mg d'équivalents ASA.

Il est à noter qu'il peut être intéressant de mélanger dans une même gélule, un même comprimé ou une même poudre, au moins deux types de microcapsules à cinétiques d'absorption différentes mais comprises dans le cadre caractéristique de l'invention (profil courbe C de la **fig. 1**).

Selon un autre de ses aspects, l'invention est relative à une méthode de prévention et/ou de traitement des troubles pathologiques associés à des excès de thromboxane et, en particulier, des maladies et risques cardiovasculaires. Cette méthode consiste à administrer, per os, les microcapsules et/ou les formes galéniques selon l'invention, de préférence par prise unique journalière, à une dose comprise entre 75 et 320 mg d'équivalents ASA.

Il ressort de ce qui précède que les microcapsules de l'invention sont très efficaces sur le plan pharmacologique, parfaitement tolérables par l'organisme, notamment au niveau gastrique, conditionnables sous diverses formes galéniques appropriées et, enfin, aisées et peu onéreuses à obtenir.

L'invention sera mieux comprise à la lumière des exemples ci-après, donnés uniquement à titre d'illustration et permettant de bien comprendre l'invention et de faire ressortir ses variantes de réalisation et/ou de mise en oeuvre, ainsi que ses différents avantages.

### EXEMPLES

### EXEMPLE 1 : PRÉPARATION DE MICROCAPSULES À BASE D'ASPIRINE PAR ENCAPSULATION DANS UN GRANULATEUR À LIT FLUIDISÉ.

### 1.1 - MICROCAPSULES M₁ :

Pour l'enrobage de 160 mg de microparticules d'aspirine, 21,5 mg d'éthyl-cellulose (normes USPXXII), 2 mg de polyvidone (Pharmacopée française Xe édition) et 2 mg d'huile de ricin (Pharmacopée française Xe édition) sont dissous dans 311 mg d'acétone. On ajoute à la solution obtenue 208 mg d'isopropanol. Ensuite, 3 mg de stéarate de magnésium sont mis en suspension dans la solution. Le mélange résultant est mis sous agitation, qui sera maintenue pendant toute l'opération d'enrobage qui va suivre.

2 110 g de microparticules d'aspirine (granulométrie moyenne 150 µm) sont chargées dans l'appareil à lit fluidisé GLATT GPCG 3, et mises en fluidisation par un débit de 1,16 à 2 m³/min. La température de l'air à l'entrée du lit fluidisé est de 55° C et maintenue constante.

7 219 g de la suspension d'enrobage décrite ci-dessus, maintenue sous agitation constante, sont envoyés, par l'intermédiaire d'une pompe péristaltique, à une buse d'injection de 1,2 mm de diamètre et pulvérisé en continu sur les microparticules, à une pression de pulvérisation de 2,8 x 10⁵ Pa.

Après une phase de préchauffage de 10 min environ, le débit de la pompe péristaltique est réglé pour permettre la pulvérisation de 30 g de suspension d'enrobage par minute.

Les microcapsules sont alors séchées durant 15 à 30 min, à un débit de fluidisation réduit (1,16 m³/min). Elles sont ensuite sorties de la cuve et étalées sur des plateaux, qui sont mis à l'étuve, à une température de 50° C pendant environ 1 heure.

### 1.2 - MICROCAPSULES M₂:

2 100 g de microparticules d'ASA, de taille comprise entre 300 et 500 µ, sont pelliculées dans l'appareil GLATT GPCG 3, comme décrit ci-dessus pour M₁, par une suspension d'enrobage présentant la composition suivante :

| | |
|---|---|
| - EUDRAGIT RS 100 | 362,8 g |
| - Phtalate de dibutyle | 36,2 g |
| - Talc micronisé | 107,1 g |
| - Hydroxypropyl méthyl cellulose | 18,4 g |
| - Acétone2 | 902,0 g |
| - Isopropanol4 | 353,0 g |

### 1.3 - MICROCAPSULES M₃ :

2 110 g de microparticules d'ASA, de taille comprise entre 300 et 500 µm, sont pelliculées dans l'appareil GLATT GPCG 3, comme décrit ci-dessus pour M₁, par une suspension d'enrobage présentant la composition suivante :

| | |
|---|---|
| - Zéine | 308,7 g |
| - Triacétate de glycérol | 30,9 g |
| - Talc micronisé | 92,6 g |
| - Stéarate de magnésium | 58,9 g |
| - Polyvinylpyrrolidone | 30,9 g |
| - Chlorure de méthylène | 3 087,0 g |
| - Méthanol | 3 087,0 g |

### EXEMPLE 2 : PRÉPARATION D'UNE FORME PHARMACEUTIQUE À BASE DES MICROCAPSULES D'ASA SELON L'INVENTION.

Les microcapsules M₁ obtenues selon le procédé décrit dans l'exemple 1 sont ensuite mélangées avec de la silice colloïdale et du talc.

Le mélange ainsi obtenu est ensuite réparti dans des gélules de taille 0 ou 1 ou 2, en fonction du dosage unitaire recherché (320, 160 ou 80 mg).

La géluleuse utilisée est une Zanussi L 264 de capacité maximale de 5 000 unités par heure. Les gélules ont été préalablement contrôlées (couleur, quantité) et les unités contrôlées pendant la mise en gélule. Le produit final est alors contrôlé selon les spécifications nouvelles définies et adaptées à chaque produit : aspect, poids moyen, dosage, désintégration, dissolution, dosage des impuretés.

### EXEMPLE 3 : DÉTERMINATION ANALYTIQUE DE LA VITESSE DE DISSOLUTION DES MICROCAPSULES.

Le contrôle de la dissolution in vitro des microcapsules, gélules ou comprimés, a été réalisé selon les indications de la Pharmacopée européenne IIe édition intitulées : "Essai de la dissolution des formes orales solides". L'appareil à panier a été utilisé pour les formes finies (gélules et comprimés). L'appareil à panier a été choisi pour la mesure de la dissolution des microcapsules. L'appareil à panier est constitué par un récipient cylindrique, un agitateur et un bain thermostaté. Le milieu de dissolution est un tampon phosphate à pH 7,2 préparé selon la recommandation Pharmacopée. Une solution témoin d'aspirine est réalisée en dissolvant 61 mg de principe actif dans le milieu à pH 7,2 et complété à 20 ml. L'étude est réalisée dans 900 ml du milieu de dissolution à 37° C ± 0,5 et une vitesse de rotation de 100 tours par minute. L'équivalent d'une gélule est introduite dans le milieu de dissolution et 10 ml du milieu sont prélevés à 0, 1, 2, 4, 6, 8, 12 et 24 heures après le début de la manipulation. Etant donné la possibilité d'hydrolyse de l'ASA en SA, la quantité d'aspirine dissoute est déterminée par mesure de l'absorbance au point isobestique de l'ASA et du SA à 265 nm.

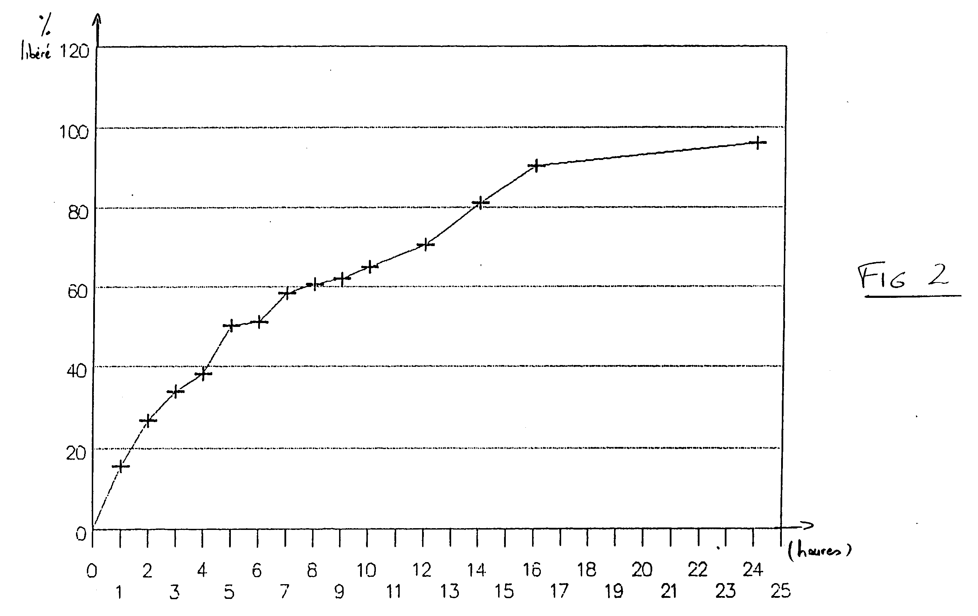

La **fig. 2** montre le pourcentage d'aspirine libérée en fonction du temps pour les microcapsules M₁ de l'exemple 1, réalisé sur un lot de 40 kg.

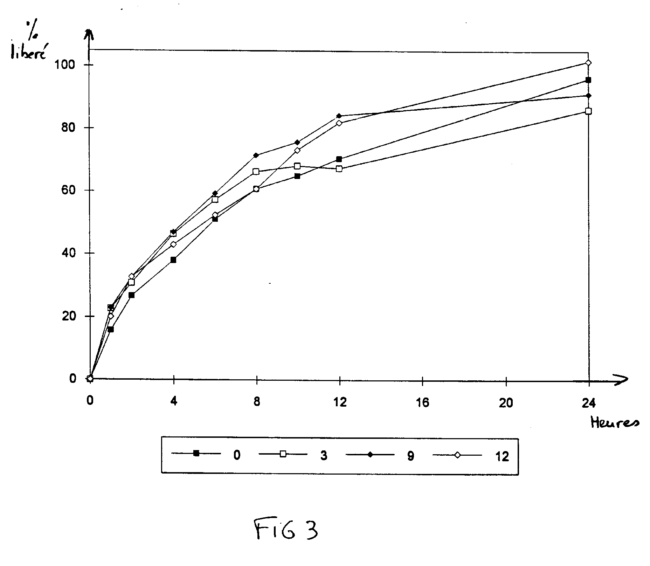

La **fig. 3** montre le profil de libération, dans les conditions décrites ci-dessus, déterminé après 0, 3, 9 et 12 mois de stabilité.

### EXEMPLE 4 : ETUDE TOXICOLOGIQUE DE L'ASPIRINE ENCAPSULÉE À LIBÉRATION CONTRÔLÉE (ALC).

On a procédé à l'étude toxicologique des microcapsules M₁ d'aspirine préparées selon l'exemple 1 comparativement à de l'aspirine commerciale (AC), sous forme de poudre blanche. La toxicité aiguë de ces deux formes pharmaceutiques d'aspirine a été évaluée et comparée par voie orale chez le rat.

### a) MÉTHODES :

Chacune des deux formulations du produit a été administrée par voie orale à des groupes de 10 rats Sprague-Dawley (5 mâles et 5 femelles) sous un volume de 10 ml/kg et en suspension dans de la carboxyméthylcellulose à 0,5 %. L'aspirine à libération contrôlée selon l'invention a été administrée à la dose de 2 500 mg/kg et l'aspirine commerciale aux doses de 740, 1 110, 1 670 et 2 500 mg/kg. Tous les animaux ont été mis à la diète hydrique avant le traitement.

La mortalité, le comportement général et l'évolution pondérale des animaux survivants ont été suivis pendant une période de 14 jours après l'administration unique du produit. Un examen anatomopathologique a été effectué sur chacun des animaux trouvés morts ou sacrifiés en fin d'étude.

La DL 50 a été calculée par la méthode de Finney.

### b) RÉSULTATS :

* AC:

Après administration du produit AC aux doses de 740, 1 110, 1 670 et 2 500 mg/kg, une baisse de l'activité spontanée en relation avec la dose administrée et une piloérection ont été observées pendant les heures qui suivent le traitement. Une baisse légère de la prise pondérale, en relation avec la dose administrée, a été notée entre le jour 1 et le jour 5, sans conséquence par la suite jusqu'à la fin de l'étude.

A l'autopsie des animaux trouvés morts en cours d'étude, aucune anomalie macroscopique n'a été notée aux doses de 740 et 1 110 mg/kg. Chez les animaux trouvés morts dans les heures qui suivent l'administration des doses de 1 670 et 2 500 mg/kg, une coloration anormalement noirâtre du foie, une décoloration avec un épaississement de la paroi au niveau du tractus digestif (intestins et estomac) et la présence de pétéchies rougeâtres au niveau de l'estomac ont été notés. Ces signes sont caractéristiques d'une intolérance gastrique prononcée.
* ALC:

Le comportement général et l'évolution pondérale des animaux traités avec ALC à la dose de 2 500 mg/kg sont normaux pendant toute la durée de l'étude.

L'autopsie des animaux trouvés morts ou sacrifiés en fin d'étude n'a mis en évidence aucune anomalie macroscopiquement visible.

### c) CONCLUSION :

Dans les conditions expérimentales, la DL 50 du produit AC, administré par voie orale chez le rat, est de 1 432 mg/kg. Les limites inférieure et supérieure de l'intervalle de confiance, pour un seuil de probabilité de 95 %, étaient, respectivement, de 936 mg/kg et de 2 394 mg/kg. La DL 0 du produit ALC selon l'invention, administré par voie orale chez le rat, était supérieure ou égale à 2 500 mg/kg, dose à laquelle aucune anomalie du comportement ou d'anomalie au niveau des principaux organes n'ont été observées.

### EXEMPLE 5 : ETUDE PHARMACOCINÉTIQUE DE L'ASPIRINE ENCAPSULÉE À LIBÉRATION CONTRÔLÉE SUR 32 HEURES.

Des essais ont été réalisés chez l'homme, afin de déterminer la biodisponibilité des microcapsules selon l'invention et de vérifier le degré d'inhibition de la cyclooxygénase plaquettaire par la mesure du thromboxane B₂ sérique.
Pour plus de commodité, on utilisera ci-après les abréviations suivantes :
- ALC : aspirine encapsulée à libération contrôlée selon l'invention (M₁ de l'exemple 1),
- ASA : acide acétylsalicylique,
- SA : acide salicylique.

L'étude de pharmacocinétique a été réalisée sur douze volontaires du sexe masculin ne présentant aucune affection hémato-biochimique, hémorragique, allergique ou gastro-intestinale, ne fumant pas plus de 10 cigarettes par jour et n'ayant pas participé à un essai thérapeutique ou fait don de sang dans les trois mois précédant l'étude. Ces sujets n'avaient reçu aucun médicament dans les 15 jours précédant l'étude.

Chaque sujet a reçu 2 gélules d'ALC avec 250 ml d'eau, chaque gélule correspondant à 160 mg d'ASA, ou 2 sachets d'ASPEGIC (1 sachet dosé à 250 mg et un sachet dosé à 100 mg, soit 350 mg d'ASA).

Les gélules ou sachets étaient absorbés à jeûn d'au moins 10 heures.

Un repas a été servi 4 heures après l'administration du médicament.

### a) DÉTERMINATION DU PRINCIPE ACTIF DANS LE PLASMA :

Des échantillons de sang ont été prélevés chez les sujets avant l'administration du médicament et aux temps 0,5, 1, 1,5, 2, 3, 4, 5, 6, 8, 12, 16, 24 et 32 heures après le traitement.

Le dosage de l'ASA et du SA dans le plasma a été effectué selon une méthode adaptée de celle de R. JAMES et al. (R. JAMES et al., J. Chrom. Biomed. Appl., 1984, 310, 343-352) et résumée ci-après.

A 0,5 ml de plasma contenant du fluorure de potassium pour éviter l'hydrolyse de l'aspirine sont ajoutés 50 µl d'une solution d'étalon interne (3, 4, 5-triméthoxy-benzaldéhyde, 25 µg/ml) et 50 µl d'acide perchlorique à 30 %.

Le mélange est agité 30 secondes au vortex et centrifugé pendant 5 min à 3 000 t/min. Environ 20 µl de la phase surnageante sont utilisés pour l'analyse chromatographique.

La séparation est effectuée sur une colonne Lichrospher 100 RP-18, 5 µm, 250 x 4 mm, (Merck) avec une phase mobile Acétonitrile/Méthanol/H₃PO₄ 0,085 % (10/40/50, v/v/v). Le débit de la phase mobile est fixé à 1 ml/min et la détection est réalisée en UV à 230 nm.

Les concentrations plasmatiques moyennes en ASA et en SA ont été déterminées et rassemblées dans le tableau 1 ci-après.

**TABLEAU 1**

| **CONCENTRATIONS PLASMATIQUES MOYENNES EN ASA ET SA** | | | | |
|---|---|---|---|---|
| TEMPS (h) | Cpm ASA (µg/ml) | | Cpm SA (µg/ml) | |
| | ALC | ASPEGIC | ALC | ASPEGIC |
| 0 | 0 | 0 | 0 | 0 |
| 0,5 | 0,59 | 5,88 | 1,11 | 18,9 |
| 1 | 0,62 | 1,31 | 2,60 | 18,6 |
| 1,5 | 0,44 | 0,43 | 3,27 | 17,0 |
| 2 | 0,29 | 0,18 | 3,41 | 14,3 |
| 3 | 0,07 * | nq | 3,49 | 11,7 |
| 4 | nq | nq | 3,20 | 9,05 |
| 5 | nq | nd | 2,85 | 7,10 |
| 6 | nd | nd | 2,43 | 5,28 |
| 8 | nd | nd | 1,84 | 2,87 |
| 12 | nd | nd | 1,34 | 0,94 |
| 16 | nd | nd | 1,13 | 0,25 |
| 24 | nd | nd | 0,83 | nq |
| 32 | nd | nd | 0,43 | nq |
| - Cpm : concentration plasmatique moyenne, - nq : non quantifiable, - nd : non détecté, - *: sous la limite de sensibilité | | | | |

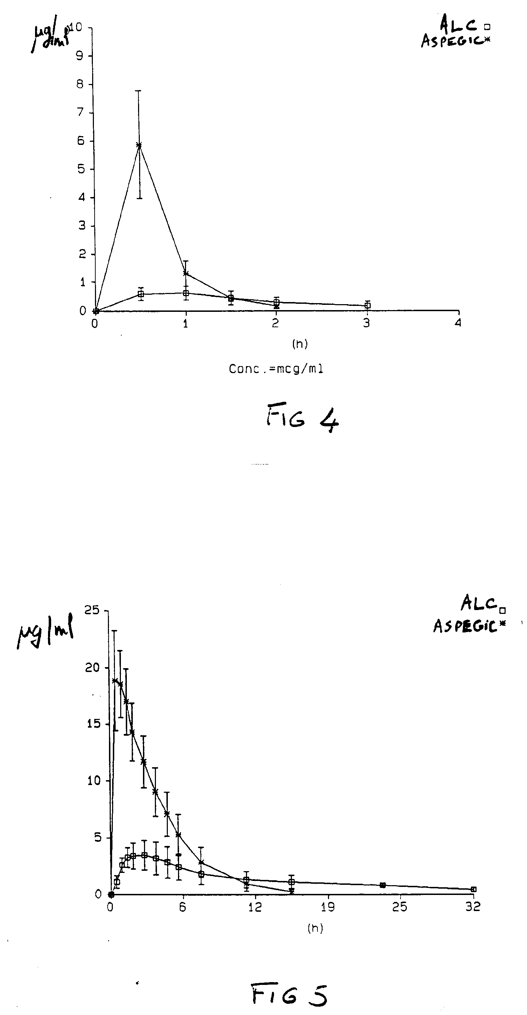

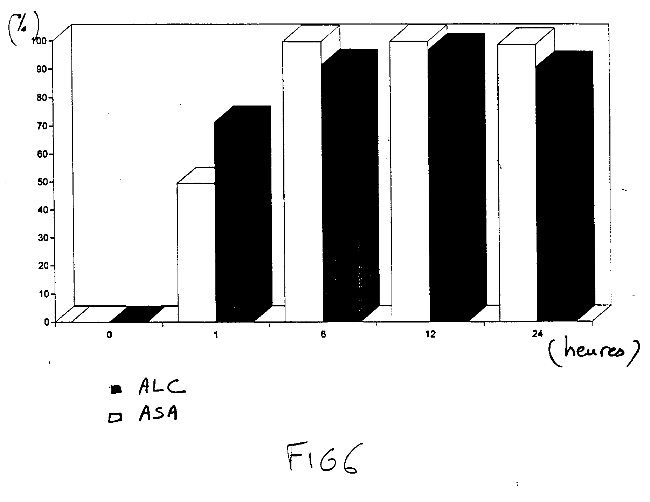

Ces résultats sont illustrés sur les **fig. 4** et **5** annexées, qui représentent l'évolution des concentrations plasmatiques moyennes en microgrammes/ml, respectivement en ASA et SA, en fonction du temps (ALC et témoin ASPEGIC).

L'acide acétylsalicylique est détectable dans le plasma jusqu'au temps 2 heures puis aux temps 4 et 5 heures, à des concentrations très faibles, inférieures ou égales à 0,25 µg/ml. L'acide salicylique est détectable très rapidement, à des concentrations qui s'élèvent jusqu'à atteindre un pseudo-plateau (de 0,8 à 1,8 µg/ml) entre la 8ème et la 24ème heure.

Les AUC (aires sous la courbe) montrent que la biodisponibilité relative de l'ALC par rapport à l'ASPEGIC est d'environ 72 %. De plus, le SA n'est détectable que jusqu'à la 16ème heure après administration d'ASPEGIC alors qu'il l'est encore après la 32ème heure après administration d'ALC.

Ces résultats montrent que les microcapsules selon l'invention permettent d'obtenir une cinétique de libération contrôlée telle que l'acide acétylsalicylique est entièrement désacétylé lors du premier passage hépatique, ce qui conduit à l'inhibition de la cyclooxygénase plaquettaire, en laissant intacte l'activité de la cyclooxygénase périphérique.

### b) DOSAGE SÉRIQUE DU THROMBOXANE B₂ (ALC UNIQUEMENT) :

Des échantillons de sang ont été prélevés chez les sujets aux temps 1, 2, 3, 4, 5, 6, 8, 12, 16, 24 et 32 heures après le traitement. Les échantillons sont placés au bain-marie (37° C) pendant 1 heure, puis centrifugés. Le sérum est ensuite prélevé, réparti en 2 tubes qui sont congelés jusqu'au moment de l'analyse.

La méthode de mesure du Thromboxane B₂ est un dosage enzymo-immunologique utilisant des anticorps spécifiques contre le thromboxane et un traceur enzymatique correspondant, le Thromboxane B₂ couplé à l'acétylcholinestérase.

Il s'agit d'un dosage par compétition utilisant des plaques de microtitration à 96 puits recouverts d'immunoglobulines monoclonales de souris anti IgG de lapin. L'anticorps spécifique anti-thromboxane B₂, le standard ou l'échantillon biologique et le traceur sont ajoutés dans des volumes de 50 µl.

Les réactions et dilutions se font dans un tampon phosphate contenant de l'albumine. Après une nuit d'incubation à 4° C, les plaques sont lavées, le substrat enzymatique contenant le réactif d'Ellman est alors distribué dans chaque puits. Après agitation pour le développement de la coloration, l'absorbance est mesurée à 414 nm au bout de 2 heures à l'aide d'un spectrophotomètre.

La coloration développée est proportionnelle à la quantité de Thromboxane B₂ présente dans l'échantillon. Les résultats obtenus sont rassemblés dans le Tableau 2 ci-après :

**TABLEAU 2**

| **CONCENTRATION SÉRIQUE EN THROMBOXANE B**_{**2**} | | |
|---|---|---|
| TEMPS (h) | CONCENTRATION (ng/ml) | % INHIBITION |
| 0 | 300,53 | - |
| 1 | 91,65 | 69,5 |
| 2 | 56,76 | 81,1 |
| 3 | 34,29 | 88,6 |
| 4 | 26,60 | 91,1 |
| 5 | 31,72 | 89,4 |
| 6 | 25,44 | 91,5 |
| 8 | 12,43 | 95,9 |
| 12 | 12,61 | 95,4 |
| 16 | 19,51 | 93,5 |
| 24 | 28,90 | 90,4 |
| 32 | 35,35 | 88,2 |

L'inhibition maximale après administration de 320 mg d'aspirine encapsulée selon l'invention est obtenue au bout de 8 heures et demeure élevée (88,2 %) 32 heures après l'administration.

La concentration sérique en Thromboxane B₂ chute rapidement, preuve que la formulation selon l'invention libère effectivement de l'ASA que l'on ne retrouve pas, en revanche, dans la circulation générale.

Ceci confirme que l'administration des microcapsules à libération contrôlée, à base d'aspirine selon l'invention, permet d'inhiber la cyclooxygénase plaquettaire.

### EXEMPLE 6 : ETUDE PHARMACOCINÉTIQUE ET DE PHARMACOLOGIE CLINIQUE DE L'ASA MICROENCAPSULÉE ET ENROBÉE SELON L'INVENTION, APRÈS ADMINISTRATION RÉPÉTÉE, PENDANT 28 JOURS ET COMPARAISON AVEC UNE FORME CONVENTIONNELLE.

Douze volontaires sains, de sexe masculin, ont été affectés à l'un ou l'autre des deux traitements suivants, selon un plan de randomisation obtenu par tirage au sort. Le groupe A a reçu une gélule de 320 mg d'ASA encapsulée (microcapsules M₁ de l'exemple 1) par voie orale chaque matin pendant 28 jours. Le groupe B a reçu 350 mg d'ASA témoin non encapsulée en solution chaque matin pendant 28 jours. Dans l'un et l'autre cas, les sujets ont absorbé les traitements avec 200 ml d'eau. Les sujets ont été répartis dans trois groupes débutant l'étude à des dates différentes. Lors de chaque période, des prélèvements sanguins (5 ml) ont été réalisés chez chacun des sujets pour le dosage de l'aspirine et de son métabolite, l'acide salicylique avant l'administration du traitement à J1 et J28, à J1 aux temps suivants (H0 étant l'heure précise de l'administration du traitement) : 5 min, 10 min, 15 min, H0,5, H1, H2, H3, H4, H5, H6, H8, H12, H16, à J5 et J14 à H0,5, H1, H2, H3, H4, H5, H6, H8, H12, H16, à J28 aux temps identiques à ceux de J5 et J14 poursuivis par H24, H36, H48 et à J42 un prélèvement en début de matinée.

Des prélèvements (5 cl) pour la mesure du Thromboxane B₂ sérique ont été réalisés avant l'administration du traitement journalier à J1, J2, J3, J4, J5, J14, J21, J27, J28 et J42.

Les prélèvements sanguins pour les dosages de l'aspirine et de son métabolite, l'acide salicylique, ont été réalisés à la seringue sur un cathéter court intraveineux à J1, J5, J14 et J28 et par ponction directe avant administration du traitement journalier chaque autre jour. Le sang était immédiatement versé dans des tubes vacutainer placés dans la glace, contenant 50 µl d'héparinate de sodium (1 000 unités/ml) et 50 µl d'une solution aqueuse de fluorure de sodium à 50 % et agités doucement pendant 2 min, puis centrifugés à 4° C, 6 000 tours/min pendant 3 min. Le plasma, séparé rapidement du culot globulaire, a été transvasé dans deux tubes en verre convenablement étiquettés, puis congelés immédiatement à - 20° C.

### a) PRÉLÈVEMENTS URINAIRES :

Lors de chaque période, des prélèvements urinaires pour dosage de la créatininurie et mesures des prostaglandines plaquettaires et vasculaires ont été réalisés chez chacun des sujets.

Les urines du matin ont été recueillies à 7 heures, les jours J1, J2, J14, J27 et J28.

Il était demandé aux sujets de vider leur vessie entre 0 et 1 heure, puis de ne plus uriner jusqu'au matin, afin d'obtenir les urines de 6 heures. La miction du matin a été réalisée avant le lever. Pour chaque recueil :
- 20 ml ont été analysés afin de réaliser un dosage de créatininurie,
- 40 ml ont été transvasés dans deux tubes en polypropylène de 20 ml convenablement étiquettés, puis congelés immédiatement à - 20° C.

### b) MESURES DU TEMPS DE SAIGNEMENT :

Le temps de saignement a été mesuré à J0, puis à J27 lors de chaque période, immédiatement avant l'administration du traitement.

Méthode : le temps de saignement a été mesuré par la technique de Duke : incision de la peau de la face antérieure du lobe de l'oreille préalablement désinfectée à l'éther. Un chronomètre était alors déclenché. Les gouttes de sang formées ont été recueillies toutes les 30 secondes sur un buvard, sans appuyer. Le temps de saignement est le temps au bout duquel le saignement s'est arrêté.

### c) TOLÉRANCE GASTRIQUE :

A J28, lors de chaque période, la tolérance gastrique a été évaluée par une gastroscopie.

En cas d'observation d'une ou plusieurs lésions importantes, le sujet devait être exclu de l'étude.

L'analyse de l'aspirine et de l'acide salicylique a été réalisée en utilisant une méthode HPLC spécialement développée pour le dosage des faibles taux dans les milieux biologiques. L'analyse du Thromboxane B₂ sérique a été effectuée en utilisant une méthode de dosage immunoenzymatique décrite par PRADELLES & coll. [Analytical Chemistry 57, 1170-1173 (1985) ; Ann. Biol. Clin. 43, 475-484 (1985)]. Pour les échantillons urinaires, l'analyse du 11 déhydro Thromboxane B₂, dinor ThromboxaneB₂, dinor-6-céto prostaglandine F1α a été réalisée par la méthode ci-dessus citée, avec extraction préalable en phase solide et purification par chromatographie sur couche mince. Cette méthode a été validée par chromatographie gazeuse couplée à la spectrométrie de masse utilisant une détection en ionisation ionique négative par LELLOUCHE et coll. [Prostaglandine 40, 297-310 (1990)].

La **fig. 6** montre le pourcentage d'inhibition de la production de Thromboxane B₂ sérique par l'ASA encapsulée (ALC) et par l'ASA témoin.

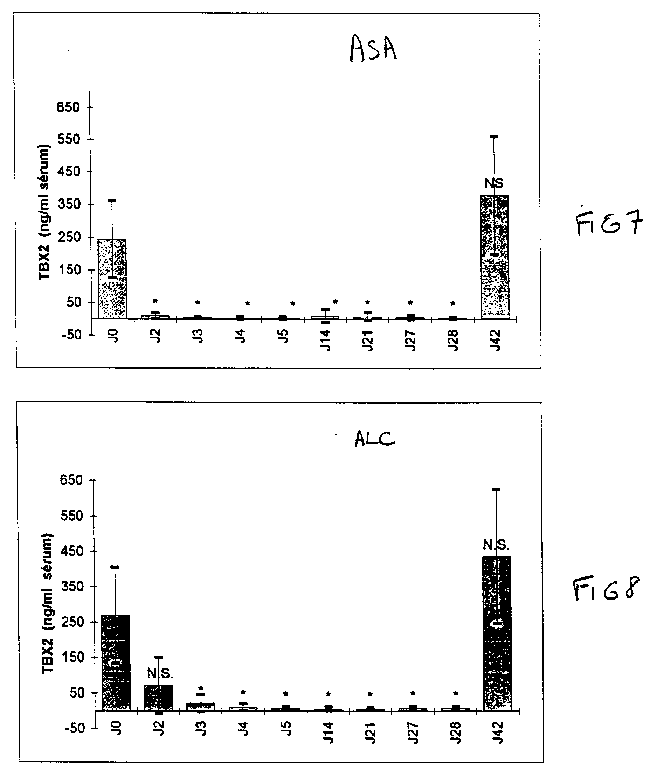

Les **fig. 7** et **8** montrent l'inhibition du Thromboxane B₂, aux jours J0, J1, J2, J3, J4, J5, J14, J27, J28 et J42 par l'ASA témoin et l'ALC, respectivement. Sur chaque rectangle de l'histogramme, on représente l'écart type de concentration. Le signe * marque le caractère significatif, sur le plan statistique, de la chute du taux de thromboxane par rapport au jour J0 témoin. Ces indications conventionnelles sont également utilisées sur certaines des autres figures ci-après.

Le Thromboxane B₂ est un métabolite du Thromboxane A₂, dont la synthèse est catalysée par la cyclooxygénase plaquettaire.

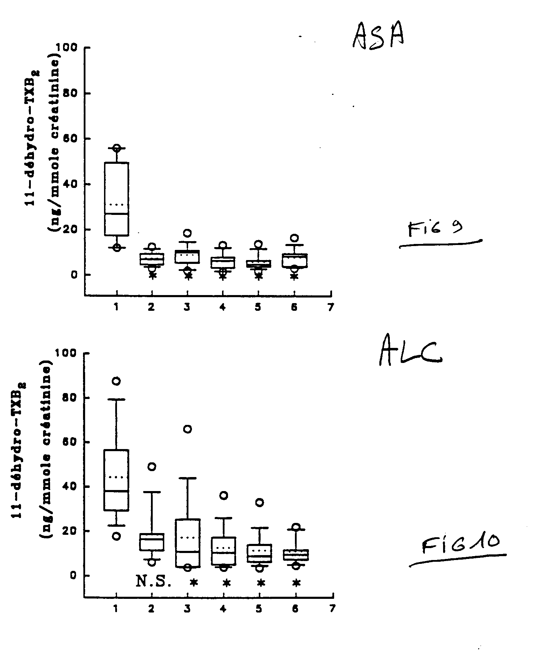

Les **fig. 9** et **10** montrent l'inhibition de la production de 11 déhydro-Thromboxane B₂, par l'ASA témoin et l'ALC, respectivement, à différents temps de mesure : J0, J1, J14, J21, J27 et J28, correspondant aux numéros 1 à 7 en abscisse.

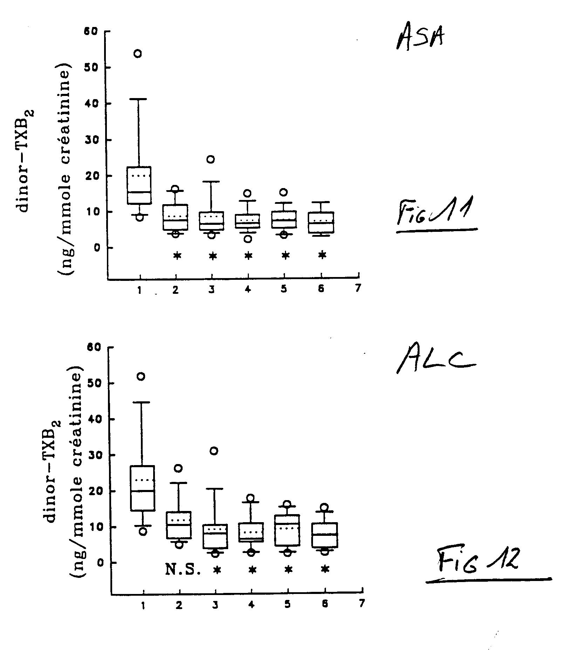

Les **fig. 11** et **12** montrent l'inhibition de la production de dinor-Thromboxane B₂ urinaire par l'ASA témoin et l'ASA encapsulée (ALC), respectivement, à différents temps de mesure : J0, J1, J14, J21, J27 et J28, correspondant aux numéros 1 à 7 en abscisse.

Ce sont deux métabolites urinaires du Thromboxane B₂, bien que 20 % de l'excrétion urinaire du 2-3 dinor-Thromboxane ne soit pas d'origine plaquettaire.

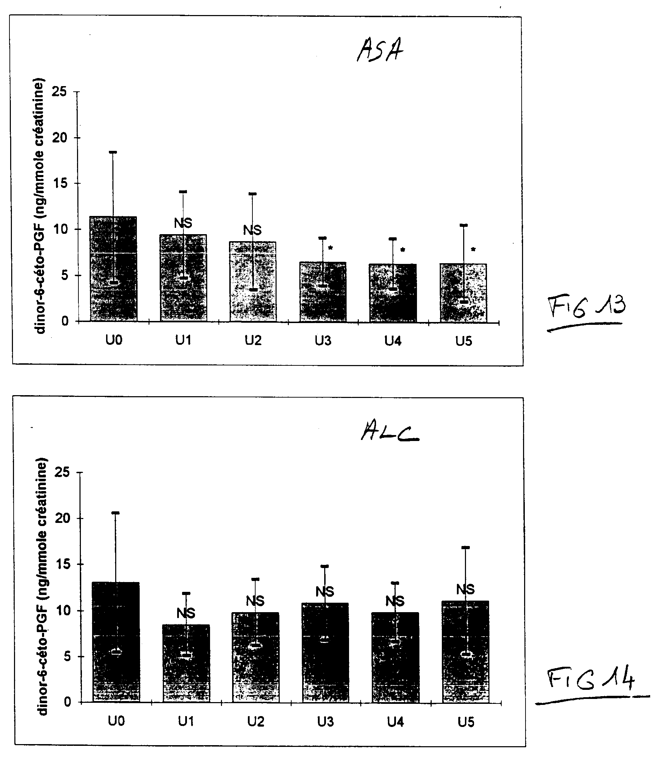

Les **fig. 13** et **14** démontrent l'effet de l'ASA témoin et de l'ASA encapsulée (ALC), respectivement, sur l'inhibition de dinor-6-céto prostaglandine F1α urinaire et ce, pour chacun des temps de recueil urinaire à : J0, J1, J14, J21, J27 et J28, correspondant aux numéros U0 à U5 en abscisse.

Le 2-3-dinor-6-céto prostaglandine F1α est le métabolite urinaire de la prostacycline (prostaglandine I2) d'origine vasculaire et gastrique.

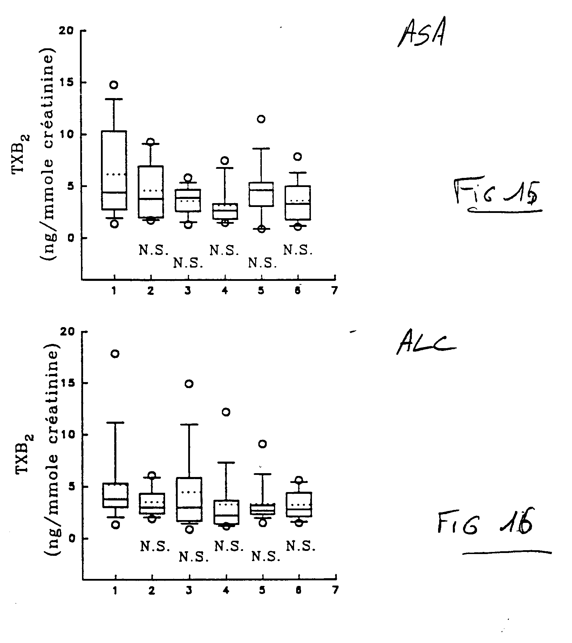

Les **fig. 15** et **16** montrent l'influence des traitements à l'aide d'ASA témoin et d'ASA encapsulée (ALC), respectivement, sur l'excrétion urinaire du Thromboxane B₂ aux temps : J0, J1, J14, J21, J27 et J28, correspondant aux numéros 1 à 7 en abscisse.

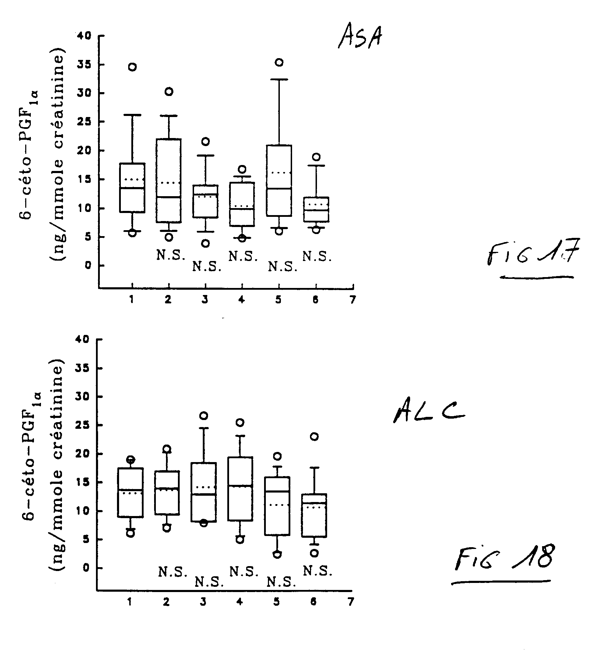

Les **fig. 17** et **18** montrent l'influence des traitements à l'aide d'ASA témoin et d'ALC, respectivement, sur l'excrétion urinaire de la 6-céto prostaglandine F1α, aux temps : J0, J1, J14, J21, J27 et J28, correspondant aux numéros 1 à 7 en abscisse. Le thromboxane B₂ et la 6-céto prostaglandine F1α sont des métabolites du Thromboxane B₂ et prostaglandine F1α d'origine rénale.

La comparaison des résultats et des figures ci-dessus indiqués (**fig. 6 à 18**) montre à l'évidence que l'ASA encapsulée a le même pouvoir inhibiteur que l'ASA témoin sur le Thromboxane d'origine plaquettaire : 96,93 % vs 98,15 % dès le troisième jour de traitement. Les résultats sont confirmés par la mesure des métabolites urinaires (77,8 % vs 75,6 % et 51,9 % vs 68,9 %).

Inversement, l'ASA encapsulée démontre une inhibition beaucoup plus faible que l'ASA témoin sur la prostacycline (8,9 % vs 43,2 %) et les prostaglandines d'origine rénale (23,8 % vs 42,8 % et 16,6 % vs 34,6 %).

Ainsi, même une dose maximale de 320 mg d'ASA encapsulée selon l'invention démontre une biosélectivité marquée sur les prostaglandines d'origine plaquettaire. Une dose de 80 à 320 mg, de préférence 160 mg, qui délivre l'aspirine à un taux de 10 mg à 40 mg/heure, de préférence 20 mg les cinq premières heures, puis 2 mg à 8 mg/h les 19 heures suivantes, de préférence 4 mg/h inhibe le Thromboxane d'origine plaquettaire d'au moins 95 % et protège la prostacycline vasculaire à plus de 90 %.

L'invention ainsi décrite résout de manière optimale le dilemme de l'aspirine et assure une protection optimale du risque d'agrégation plaquettaire d'au moins 24 heures après administration d'une seule dose quotidienne.

## Revendications

1. Microcapsules pour la libération contrôlée d'acide acétylsalicylique (ASA) en milieu gastrointestinal,
caractérisées en ce qu'elles sont constituées par des particules d'acide acétylsalicylique de taille comprise entre 100 et 1 000 µm, enrobées et conçues de telle sorte que, ingérées per os en une seule prise d'une dose D d'ASA comprise entre 75 et 320 mg, elles induisent une cinétique d'absorption d'ASA moyenne in vivo chez l'homme, s'étendant sur au moins 24 heures, ladite absorption d'ASA étant :
- inférieure ou égale à 10 % de la fraction absorbée de D à un temps **t** après l'ingestion égal à 0,4 heure,
- inférieure ou égale à 50 % en poids de la fraction absorbée de D, à **t** = 3,9 heures,
- et inférieure ou égale à 90 % en poids de la fraction absorbée de D, à **t** = 23 heures,
**t** étant donné à ± 10 % près.

2. Microcapsules selon la revendication 1, caractérisées en ce que l'absorption se déroule sur une période comprise entre 24 et 48 heures, de la façon suivante :
- 10 % de la fraction absorbée de D à **t** compris entre 0,4 et 5 heures,
- 50 % de la fraction absorbée de D à **t** compris entre 3,9 et 25 heures,
- 90 % de la fraction absorbée de D à **t** compris entre 23 et 45 heures.

3. Microcapsules selon la revendication 1, caractérisées en ce que les particules d'ASA, utilisées pour l'enrobage, ont une taille comprise entre 250 et 800 µm, de préférence entre 300 et 500 µm.

4. Microcapsules selon l'une quelconque des revendications 1 à 3, caractérisées en ce que l'enrobant représente de 5 à 50, de préférence 10 à 40 et, plus préférentiellement encore, de 10 à 35 % en poids par rapport à la masse totale desdites microcapsules.

5. Microcapsules selon l'une des revendications 1 à 4, caractérisées en ce que l'enrobant est constitué par une composition d'enrobage comprenant :
- au moins un polymère filmogène (P₁) insoluble en milieu gastro-intestinal,
- au moins un polymère (P₂) soluble dans l'eau,
- au moins une charge lubrifiante solide,
- et au moins un plastifiant hydrophobe.

6. Microcapsules selon l'une quelconque des revendications 1 à 5, caractérisées en ce que la composition d'enrobage se définit quantitativement de la façon suivante, exprimée en % en poids sur sec :
- P₁: 60 à 85, de préférence 70 à 80 %,
- P₂ : 2 à 20, de préférence 5 à 15 %,
- agent lubrifiant : 2 à 20, de préférence 8 à 20 %,
- plastifiant : 2 à 20, de préférence 5 à 15 %.

7. Microcapsules selon l'une quelconque des revendications 1 à 6, caractérisées en ce que P₁ est sélectionné parmi les produits suivants : la zéine, l'éthylcellulose, le chlorure de vinyle, l'acétate de vinyle et/ou ses copolymères, les copolymères à base d'acrylate et/ou de méthacrylate d'éthyle et/ou de méthyle et les mélanges de tous ces produits.

8. Microcapsules selon l'une quelconque des revendications 1 à 7, caractérisées en ce que P₂ est sélectionné parmi les produits suivants :
- la polyvinylpyrrolidone,
- les dérivés cellulosiques solubles dans l'eau, tels que l'hydroxypropylcellulose, l'hydroxypropylméthylcellulose, l'hydroxyméthyléthylcellulose et la méthylcellulose,
- les copolymères de l'acétate de vinyle et de l'acide crotonique,
- les copolymères de l'anhydride maléique et du méthylvinyléther,
- ainsi que parmi les mélanges et les dérivés des susdits produits.

9. Microcapsules selon l'une quelconque des revendications 1 à 8, caractérisées en ce que la charge lubrifiante solide est choisie parmi les produits suivants : les sels alcalinoterreux de l'acide stéarique, les silicates de magnésium, le kaolin, la silice, le talc et leurs mélanges.

10. Microcapsules selon l'une quelconque des revendications 1 à 9, caractérisées en ce que le plastifiant est constitué par au moins l'un des composés suivants : les stéarates de glycérol, les phtalates, les citrates, les sébaçates, les esters de l'alcool cétylique, l'huile de ricin, la cutine et les résines synthétiques.

11. Microcapsules selon l'une quelconque des revendications 1 à 10 caractérisées en ce que la composition d'enrobage comprend 74 ± 2 % d'éthylcellulose, 10 ± 2 % de stéarate de magnésium, 8 ± 2 % de polyvinylpyrrolidone et 8 ± 2 % d'huile de ricin, les pourcentages étant exprimés en poids.

12. Microcapsules selon l'une quelconque des revendications 1 à 11, caractérisées en ce qu'elles sont mélangées avec 0,5 à 5 % et, de préférence, 1,5 % d'au moins un agent anti-agglomérant formé, de préférence, par du talc, de la silice colloïdale ou par un mélange des deux.

13. Procédé pour l'obtention des microcapsules selon l'une quelconque des revendications 1 à 12, caractérisé en ce qu'il consiste, essentiellement, à :
a) - préparer la composition d'enrobage par mélange de P₁, P₂, de l'agent lubrifiant et du plastifiant dans un système solvant,
b) - appliquer le mélange composition/système solvant sur des particules d'acide acétylsalicylique,
c) - sécher les microcapsules ainsi obtenues,
d) - et éventuellement mélanger ces dernières avec au moins un agent anti-agglomérant.

14. Procédé selon la revendication 13, caractérisé en ce que le système solvant est formé par des composés sélectionnés dans la liste suivante : cétones, esters, solvants chlorés, alcools, de préférence aliphatiques, alcanes et leurs mélanges :
- les composés en C₁-C₆ étant préférés,
- et l'acétone, la méthyléthylcétone, l'acétate de méthyle ou d'éthyle, le méthanol, l'éthanol, l'isopropanol et le chlorure de méthylène étant particulièrement préférés.

15. Procédé selon la revendication 13 ou la revendication 14, caractérisé en ce que l'application du mélange composition d'enrobage/système solvant s'opère par pulvérisation sur les particules d'ASA mises en mouvement, de préférence par agitation mécanique ou par soufflage (fluidisation).

16. Utilisation des microcapsules selon l'une quelconque des revendications 1 à 12 et/ou obtenues par le procédé selon l'une quelconque des revendications 13 à 15, pour la préparation de formes galéniques d'aspirine, présentant une sélectivité biochimique d'inhibition du thromboxane par rapport aux autres prostaglandines.

17. Utilisation des microcapsules selon l'une quelconque des revendications 1 à 12 et/ou obtenues par le procédé selon l'une quelconque des revendications 13 à 15, pour la préparation de formes galéniques d'aspirine utiles comme anti-agrégants plaquettaires.

18. Utilisation des microcapsules selon l'une quelconque des revendications 1 à 12 et/ou obtenues par le procédé selon l'une quelconque des revendications 13 à 15, pour la préparation de formes galéniques d'aspirine actives dans la prévention et/ou le traitement des maladies et des risques cardio-vasculaires.

19. Nouvelles formes galéniques selon l'une quelconque des revendications 16 à 18, caractérisées en ce qu'elles se présentent sous forme de comprimés, de poudres ou gélules, dosés à 20 à 500 mg, de préférence à 50 à 400 mg et, plus préférentiellement encore, à 75 à 320 mg d'équivalents ASA.

20. Nouvelles formes galéniques selon la revendication 19, caractérisées en ce qu'elles sont administrées per os par doses journalières uniques, comprenant chacune entre 75 et 320 mg d'équivalents ASA.

## Claims

1. Microcapsules for the controlled release of acetylsalicylic acid (ASA) in the gastrointestinal environment, characterized in that they comprise particles of acetylsalicylic acid with a size of between 100 and 1000 µm which are coated and designed so that, when ingested orally in a single administration of a dose D of between 75 and 320 mg of ASA, they induce moderate ASA absorption kinetics in vivo in man, extending over at least 24 hours, said ASA absorption being:
less than or equal to 10% by weight of the absorbed fraction of D at a time t after ingestion of 0.4 hour,
less than or equal to 50% by weight of the absorbed fraction of D at t=3.9 hours, and
less than or equal to 90% by weight of the absorbed fraction of D at t=23 hours,
t being given to within ±10%.

2. Microcapsules according to claim 1 characterized in that the absorption takes place over a period of between 24 and 48 hours in the following manner:
10% of the absorbed fraction of D at t=0.4 to 5 hours,
50% of the absorbed fraction of D at t=3.9 to 25 hours, and
90% of the absorbed fraction of D at t=23 to 45 hours.

3. Microcapsules according to claim 1 characterized in that the particles of ASA used for the coating operation have a size of between 250 and 800 µm, preferably between 300 and 500 µm.

4. Microcapsules according to anyone of claim 1 or 3 the coating agent represents 5 to 50% preferably 10 to 40 and still preferably 10 to 35 % by weight , based on the total mass of said microcapsules.

5. Microcapsules according to anyone of claims 1 or 3 characterized in that the coating agent consists of a coating composition comprising:
at least one film-forming polymer (P₁) insoluble in the gastrointestinal environment,
at least one water-soluble polymer (P₂),
at least one solid lubricating filler, and
at least one hydrophobic plasticizer.

6. Microcapsules according to anyone of claims 1 to 5 characterized in that the coating composition is quantitatively defined as follows, expressed in % by dry weight:
P₁ : 60 to 85%, preferably 70 to 80 %
P₂ : 2 to 20%, preferably 5 to 15 %
lubricant: 2 to 20%, preferably 8 to 20 %
plasticizer: 2 to 20%, preferably 5 to 15 %

7. Microcapsules according to any one of claims 1 to 6 characterized in that P₁. is selected from the following products: zein, ethyl cellulose, vinyl chloride, vinyl acetate and/or its copolymers, copolymers based on ethyl and/or methyl acrylate and/or methacrylate, and mixtures of all these products.

8. Microcapsules according to anyone of claims 1 to 7 characterized in that P₂ is selected from the following products:
- polyvinylpyrrolidone,
- water-soluble cellulose derivatives such as hydroxypropyl cellulose, hydroxypropyl methyl cellulose, hydroxymethyl ethyl cellulose and methyl cellulose,
- vinyl acetate/crotonic acid copolymers,
- maleic anhydride/methyl vinyl ether copolymers, and
- mixtures and derivatives of the abovementioned products.

9. Microcapsules according to anyone of claims 1 to 8 characterized in that the solid lubricating filler is selected from the following products: alkaline earth metal salts of stearic acid, magnesium silicates, kaolin, silica, talc and mixtures thereof.

10. Microcapsules according to anyone of claims 1 to 9 characterized in that the plasticizer consists of at least one of the following compounds: glycerol stearates, phthalates, citrates, sebacates, esters of cetyl alcohol, castor oil, cutin and synthetic resins.

11. Microcapsules according to anyone of claims 1 to 10 characterized in that the coating composition comprises 74.±.2% of ethyl cellulose 10.±.2% of magnesium stearate 8.±.2% of polyvinylpyrrolidone and 8.±.2% of castor oil, the percentages being expressed by weight.

12. Microcapsules according to anyone of claims 1 to 11 characterized in that they are mixed with 0.5 to 5% preferably of at least one anticaking agent preferably formed of talc, colloidal silica or a mixture of the two.

13. A process for the preparation of microcapsules according anyone of the claims 1 to 12, characterized in that it consists,essentially, in:
a) preparing a coating composition by mixing P₁ , P₂, some lubricant and some plasticizer in a solvent system,
b) applying the composition/solvent system mixture to particles of acetylsalicylic acid, and
c) drying the resulting microcapsules.
d) and eventually, mixing these latter with at least one anti-caking agent.

14. The process according to claim 13 , characterized in that the solvent system is formed of compounds selected from the group consisting of: ketones, esters, chlorinated solvents, alcohols-preferably aliphatic-, alkanes and mixtures thereof;
C₁-C₆ compounds being preferred;
acetone, methyl ethyl ketone, methyl acetate, ethyl acetate, methanol, ethanol, isopropanol and methylene chloride being particularly preferred.

15. Process according to claim 13 or 14 , characterized in that composition/solvent system mixture is applied by being sprayed onto moving particles of ASA, said movement preferably being created by mechanical agitation or by blowing (fluidization).

16. Use of microcapsules according anyone of claims 1 to 12 and/or obtained by the process according anyone of the claims 13 to 15 , for the preparation of new galenical ASA forms, which present a biochemical selectivity for inhibiting thromboxane relative to other prostaglandins.

17. Use of microcapsules according anyone of claims 1 to 12 and/or obtained by the process according anyone of the claims 13 to 15 , for the preparation of new galenical ASA forms, useful as platelet anti-agregants.

18. Use of microcapsules according anyone of claims 1 to 12 and/or obtained by the process according anyone of the claims 13 to 15 , for the preparation of new galenical ASA forms,active in the prevention and/or the treatment of cardiovascular diseases and risks.

19. New galenical forms according anyone of claims 16 to 18, characterized in that they are presented in the form of tablets, powders or gelatin capsules , containing 20 to 500 mg , preferably 50 to 400 mg and ,still more preferably 75 to 320 mg ASA equivalents.

20. New galenical forms according to claim 19, characterized in that they are orally administrated in single daily doses , each comprising between 75 and 320 mg ASA equivalents.

## Patentansprüche

1. Mikrokapseln zur kontrollierten Freisetzung von Acetylsalicylsäure (ASA) im gastrointestinalen Milieu, dadurch gekennzeichnet, daß sie aus Acetylsalicilysäureteilchen mit einer Größe zwischen 100 und 1000 µm bestehen, die umhüllt und so entwickelt sind, daß sie oral bei einer einzigen Einnahme einer Dosis D an ASA zwischen 75 und 320 mg aufgenommen eine mittlere Kinetik der ASA-Absorption in vivo beim Menschen, die sich über mindestens 24 h erstreckt, induzieren, wobei die Absorption von ASA
- kleiner oder gleich 10 % der absorbierten Fraktion von D bei einer Zeit t nach der Einnahme von 0,4 h ist,
- kleiner oder gleich 50 Gew.-% der absorbierten Fraktion von D bei t = 3,9 h ist,
- kleiner oder gleich 90 Gew.-% der absorbierten Fraktion von D bei t = 23 h ist,
wobei t auf etwa ± 10 % angegeben ist.

2. Mikrokapsel nach Anspruch 1, dadurch gekennzeichnet, daß die Absorption über eine Zeitspanne zwischen 24 und 48 h wie folgt abläuft:
- 10 % der absorbierten Fraktion an D bei t zwischen 0,4 und 5 h,
- 50 % der absorbierten Fraktion an D bei t zwischen 3,9 und 25 h,
- 90 % der absorbierten Fraktion an D bei t zwischen 23 und 45 h.

3. Mikrokapseln nach Anspruch 1, dadurch gekennzeichnet, daß die zur Umhüllung verwendeten ASA-Teilchen eine Größe zwischen 250 und 800 µm, bevorzugt zwischen 300 und 500 µm, besitzen.

4. Mikrokapseln nach einem der Ansprüche 1 bis 3, dadurch gekennzeichnet, daß die Umhüllung 5 bis 50, bevorzugt 10 bis 40, und noch bevorzugter 10 bis 35 Gew.-% bezogen auf die Gesamtmasse der Mikrokapseln beträgt.

5. Mikrokapseln nach einem der Ansprüche 1 bis 4, dadurch gekennzeichnet, daß die Umhüllung aus einer Umhüllungszusammensetzung besteht, die umfaßt:
- mindestens ein filmbildendes Polymeres (P₁), das im gastrointestinalen Milieu unlöslich ist,
- mindestens ein wasserlösliches Polymeres (P₂),
- mindestens einen festen Gleitfüllstoff,
- und mindestens einen hydrophoben Weichmacher.

6. Mikrokapseln nach einem der Ansprüche 1 bis 5, dadurch gekennzeichnet, daß die Umhüllungszusammensetzung quantitativ wie folgt definiert ist, ausgedrückt als Gew.-% der Trockenmasse:
- P₁: 60 bis 85, bevorzugt 70 bis 80 %,
- P₂: 2 bis 20, bevorzugt 5 bis 15 %,
- Gleitmittel: 2 bis 20, bevorzugt 8 bis 20 %,
- Weichmacher: 2 bis 20, bevorzugt 5 bis 15 %.

7. Mikrokapseln nach einem der Ansprüche 1 bis 6, dadurch gekennzeichnet, daß P₁ aus den folgenden Verbindungen ausgewählt ist: Zein, Ethylcellulose, Vinylchlorid, Vinylacetat und/oder dessen Copolymere, Copolymere auf der Basis von Ethyl- und/oder Methyl(meth)acrylat und Gemische aller dieser Verbindungen.

8. Mikrokapseln nach einem der Ansprüche 1 bis 7, dadurch gekennzeichnet, daß P₂ aus den folgenden Verbindungen ausgewählt ist:
- Polyvinylpyrrolidon,
- wasserlösliche Cellulosederivate wie Hydroxypropylcellulose, Hydroxypropylmethylcellulose, Hydroxymethylethylcellulose und Methylcellulose,
- Copolymere von Vinylacetat und Crotonsäure,
- Copolymere von Maleinsäureanhydrid und Methylvinylether,
- sowie Gemische und Derivate der vorstehenden Verbindungen.

9. Mikrokapseln nach einem der Ansprüche 1 bis 8, dadurch gekennzeichnet, daß der feste Gleitfüllstoff aus den folgenden Verbindungen ausgewählt ist: Erdalkalisalze der Stearinsäure, Magnesiumsilikate, Kaolin, Kieselsäure, Talk und Gemische davon.

10. Mikrokapseln nach einem der Ansprüche 1 bis 9, dadurch gekennzeichnet, daß der Weichmacher aus mindestens einer der folgenden Verbindungen besteht: Glycerinstearate, Phthalate, Citrate, Sebacate, Cetylalkoholester, Rizinusöl, Cutin und synthetische Harze.

11. Mikrokapseln nach einem der Ansprüche 1 bis 10, dadurch gekennzeichnet, daß die Umhüllungszusammensetzung 74 ± 2 % Ethylcellulose, 10 ± 2 % Magnesiumstearat, 8 ± 2% Polyvinylpyrrolidon und 8 ± 2% Rizinusöl umfaßt, wobei die Prozentsätze als Gew.-% ausgedrückt sind.

12. Mikrokapseln nach einem der Ansprüche 1 bis 11, dadurch gekennzeichnet, daß sie mit 0,5 bis 5 % und bevorzugt mit 1,5 % mindestens eines Antiagglomerationsmittels, das bevorzugt aus Talk, colloidaler Kieselsäure oder einem Gemisch der beiden gebildet ist, vermischt sind.

13. Verfahren zur Herstellung von Mikrokapseln nach einem der Ansprüche 1 bis 12, dadurch gekennzeichnet, daß es im wesentlichen besteht aus
a) Herstellen der Umhüllungszusammensetzung durch Vermischen von P₁, P₂, dem Gleitmittel und dem Weichmacher in einem Lösungsmittelsystem,
b) Aufbringen des Gemisches Zusammensetzung/Lösungsmittelsystem auf Teilchen aus Acetylsalicylsäure,
c) Trocknen der so erhaltenen Mikrokapseln,
d) und gegebenenfalls Vermischen dieser zuletztgenannten mit mindestens einem Antiagglomerationsmittel.

14. Verfahren nach Anspruch 13, dadurch gekennzeichnet, daß das Lösungsmittelsystem aus Verbindungen, ausgewählt aus der folgenden Liste: Ketone, Ester, chlorierte Lösungsmittel, Alkohole, bevorzugt aliphatische, Alkane und ihre Gemische, gebildet wird,
- wobei die C₁-C₆-Verbindungen bevorzugt sind,
- und Aceton, Methylethylketon, Methylacetat oder Ethylacetat, Methanol, Ethanol, Isopropanol und Methylenchlorid besonders bevorzugt sind.

15. Verfahren nach Anspruch 13 oder 14, dadurch gekennzeichnet, daß das Aufbringen des Gemisches Umhüllungszusammensetzung/Lösungsmittelsystem durch Pulverisation über die bevorzugt durch mechanische Bewegung oder durch Aufwirbeln (Fluidisierung) in Bewegung gebrachten ASA-Teilchen erfolgt.

16. Verwendung von Mikrokapseln nach einem der Ansprüche 1 bis 12 und/oder erhalten durch das Verfahren nach einem der Ansprüche 13 bis 15 zur Herstellung von galenischen Aspirinformen mit einer biochemischen Selektivität zur Hemmung von Thromboxan gegenüber anderen Prostaglandinen.

17. Verwendung von Mikrokapseln nach einem der Ansprüche 1 bis 12 und/oder erhalten durch das Verfahren nach einem der Ansprüche 13 bis 15 zur Herstellung von galenischen Aspirinformen, die als Thrombozytenaggregationshemmer nützlich sind.

18. Verwendung von Mikrokapseln nach einem der Ansprüche 1 bis 12 und/oder erhalten durch das Verfahren nach einem der Ansprüche 13 bis 15 zur Herstellung von aktiven galenischen Aspirinformen zur Verhütung und/oder Behandlung von cardiovasculären Erkrankungen und Risiken.

19. Neue galenische Formen nach einem der Ansprüche 16 bis 18, dadurch gekennzeichnet, daß sie in Form von Tabletten, Pulvern oder Gelatinekapseln mit einer Dosis von 20 bis 500 mg, bevorzugt 50 bis 400 mg und noch bevorzugter 75 bis 320 mg ASA-Äquivalenten vorliegen.

20. Neue galenische Formen nach Anspruch 19, dadurch gekennzeichnet, daß sie oral in Tageseinzeldosen zu jeweils 75 bis 320 mg ASA-Äquivalenten verabreicht werden.
